(19) Europäisches Patentamt
European Patent Office
Office européen des brevets

(11) Veröffentlichungsnummer: **0 017 205**
**B1**

(12) # EUROPÄISCHE PATENTSCHRIFT

(45) Veröffentlichungstag der Patentschrift: **31.03.82**

(51) Int. Cl.³: **C 07 D 263/44**

(21) Anmeldenummer: **80101700.5**

(22) Anmeldetag: **29.03.80**

(54) **Verfahren zur Herstellung von N-Aryloxazolidin-2,4-dionen.**

(30) Priorität: **04.04.79 DE 2913522**

(43) Veröffentlichungstag der Anmeldung: **15.10.80 Patentblatt 80/21**

(45) Bekanntmachung des Hinweises auf die Patenterteilung: **31.03.82 Patentblatt 82/13**

(84) Benannte Vertragsstaaten: **BE CH DE FR GB NL**

(56) Entgegenhaltungen:
**EP-A1-0 007 415**
**DE-A-2 343 826**
**DE-A1-2 711 659**
**DE-A1-2 813 873**
**US-A-3 703 526**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 017 205 B1

BUNDESDRUCKEREI BERLIN

## Verfahren zur Herstellung von N-Aryloxazolidin-2,4-dionen

Die Erfindung betrifft ein neues Verfahren zur Herstellung von N-Aryloxazolidin-2,4-dionen durch Umsetzung eines Nitrobenzols mit einem 2-Hydroxycarbonsäureester und Kohlenmonoxid in Gegenwart von Selen und einer basischen Verbindung bei erhöhtem Druck und erhöhter Temperatur.

Oxazolidin-2,4-dione lassen sich durch Umsetzung von Isocyanaten mit 2-Hydroxycarbonsäureestern und anschließende Cyclisierung herstellen (DE-AS 1 811 843, DE-OS 2 207 576). Die hierzu benötigten Isocyanate erhält man üblicherweise aus den entsprechenden Aminen durch Umsetzung mit Phosgen und diese Amine aus entsprechenden Nitroverbindungen durch Reduktion. Das Herstellungsverfahren ist daher aufwendig, und man muß das toxische Phosgen verwenden; aus Sicherheitsgründen und zur Vermeidung von Umweltproblemen ist so ein großer apparativer Aufwand erforderlich.

Es ist weiterhin bekannt, bei erhöhter Temperatur und erhöhtem Druck aus aromatischen Nitroverbindungen, eine Hydroxylgruppe enthaltenden organischen Verbindungen und Kohlenmonoxid in Gegenwart von Selen oder Schwefel Urethane herzustellen (DE-OS 2 343 826). Als Hydroxylverbindungen werden in den Beispielen lediglich Methanol und Äthanol veranschaulicht. Auch unter den in der Beschreibung als Einzelindividuen aufgezählten Ausgangsstoffen befindet sich kein 2-Hydroxycarbonsäureester.

Es wurde nun gefunden, daß man N-Aryloxazolidin-2,4-dione der Formel

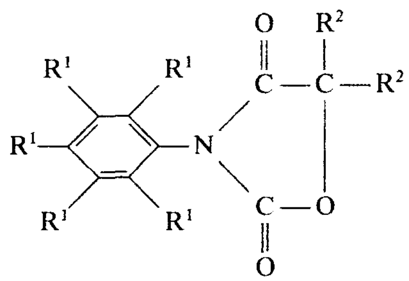

(I)

worin die einzelnen Reste $R^1$ und $R^2$ gleich oder verschieden sein können und jeweils ein Wasserstoffatom, einen aliphatischen, cycloaliphatischen, araliphatischen oder aromatischen Rest bedeuten, die beiden Reste $R^2$ darüber hinaus zusammen auch einen Methylenrest bezeichnen, $R^1$ auch jeweils für ein Halogenatom oder den Rest $-X-R^3$ steht, $R^3$ einen aliphatischen Rest bezeichnet, X ein Sauerstoffatom oder ein Schwefelatom bedeutet, vorteilhaft erhält, wenn man ein Nitrobenzol der Formel

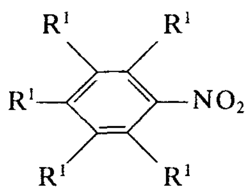

(II)

worin $R^1$ die vorgenannte Bedeutung besitzt, mit einem 2-Hydroxycarbonsäureester der Formel

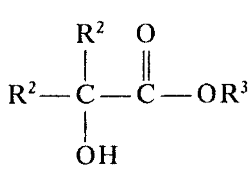

(III)

worin $R^2$ und $R^3$ die vorgenannte Bedeutung besitzen, mit Kohlenmonoxid in Gegenwart von Selen und einer basischen Verbindung bei erhöhtem Druck und erhöhter Temperatur umsetzt.

Die Umsetzung läßt sich für den Fall der Verwendung von 3,5-Dichlornitrobenzol und Vinylmilchsäuremethylester durch die folgende Formeln wiedergeben:

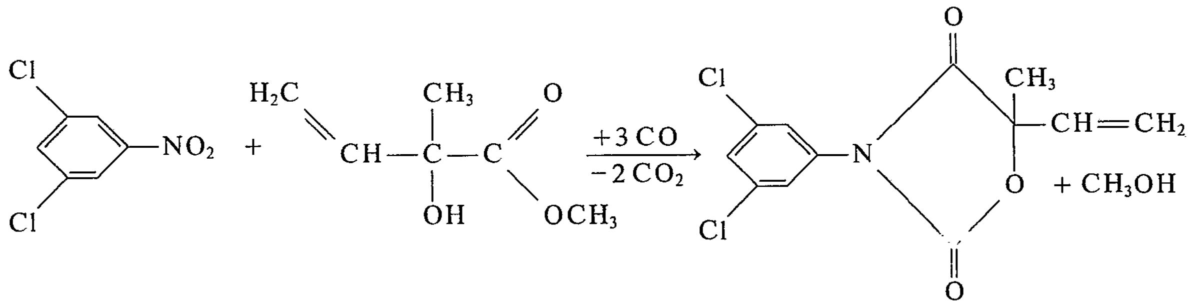


Im Vergleich zu den bekannten Verfahren liefert das Verfahren nach der Erfindung auf einfacherem und wirtschaftlicherem Wege N-Aryloxazolidin-2,4-dione in besserer Ausbeute. Alle diese vorteilhaften Ergebnisse sind im Hinblick auf den Stand der Technik überraschend. Bei der rasch verlaufenden Bildung der Endstoffe I wird ein Alkohol frei, der nach der Lehre der DE-OS 2 343 826 mit vorhandener aromatischer Nitroverbindung bevorzugt das Urethan des Alkohols ergeben sollte. Dieses Urethan wird jedoch nicht in deutlichem Maße gebildet, die Reaktion verläuft statt dessen hoch selektiv zum gewünschten Oxazolidin-2,4-dion I. Überraschenderweise beobachtet man unter den Reaktionsbedingungen auch keinerlei Polyesterbildung der 2-Hydroxycarbonsäureester durch Selbstkondensation.

Der Ausgangsstoff II wird mit dem Ausgangsstoff III in stöchiometrischer Menge oder im Überschuß eines Ausgangsstoffs bezogen auf den andern, vorteilhaft in einem Verhältnis von 1 bis 20, insbesondere 1,5 bis 10 Mol Ausgangsstoff III je Mol Ausgangsstoff II, umgesetzt. Bevorzugte Ausgangsstoffe II und III und dementsprechend bevorzugte Endstoffe I sind solche, in deren Formeln die einzelnen Reste $R^1$ und $R^2$ gleich oder verschieden sein können und jeweils ein Wasserstoffatom, einen Alkylrest mit 1 bis 7 Kohlenstoffatomen, einen Cycloalkylrest mit 5 bis 7 Kohlenstoffatomen, einen Aralkylrest oder Alkylarylrest mit 7 bis 12 Kohlenstoffatomen, einen Phenylrest bedeuten, $R^2$ auch für einen Alkenylrest mit 2 bis 7 Kohlenstoffatomen oder einen Alkoxymethylrest mit 2 bis 7 Kohlenstoffatomen steht, die beiden Reste $R^2$ darüber hinaus zusammen auch einen Methylenrest bezeichnen, $R^1$ auch jeweils für ein Chloratom, Fluoratom, Bromatom oder den Rest $-X-R^3$ steht, $R^3$ einen Alkylrest mit 1 bis 10, insbesondere 1 bis 7 Kohlenstoffatomen bezeichnet, X ein Sauerstoffatom oder ein Schwefelatom bedeutet. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Geeignete Nitrobenzole II sind z. B.: Nitrobenzol, 2-, 3- und 4-Fluornitrobenzol, 2-, 3- und 4-Chlornitrobenzol, 2-, 3- und 4-Bromnitrobenzol, 2,3-Dichlornitrobenzol, 3,4-Dichlornitrobenzol, 2,5-Dichlornitrobenzol, 3,5-Dichlornitrobenzol, 2,6-Dichlornitrobenzol, 2,3,4-Trichlornitrobenzol, 3,4,5-Trichlornitrobenzol, 2,4,6-Trichlornitrobenzol, 2,3,6-Trichlornitrobenzol, 2,3,5-Trichlornitrobenzol, 2-, 3- und 4-Methoxynitrobenzol, 2-, 3- und 4-Methylnitrobenzol; entsprechende durch Äthyl-, Propyl-, Isopropyl-, Butyl-, Isobutyl-, sek.-Butyl-, tert.-Butyl-, Cyclohexyl-, Benzyl-, Phenyl-, Äthoxy-, Propoxy-, Isopropoxy-, Butoxy-, Isobutoxy-, sek.-Butoxy-, tert.-Butoxy-, Methylthio-, Äthylthio-, Propylthio-, Isopropylthio-, Butylthio-, Isobutylthio-, sek.-Butylthio-, tert.-Butylthiogruppen untereinander gleich oder unterschiedlich substituierte Nitrobenzole; besonders bevorzugt sind 3,5-Dichlornitrobenzol, 2-, 3- und 4-Chlornitrobenzol, 2-, 3- und 4-Bromnitrobenzol, 2-, 3- und 4-Fluornitrobenzol, alle Dichlornitrobenzole, alle Trichlornitrobenzole, 2-, 3- und 4-Methoxynitrobenzol, 2-, 3- und 4-Methylnitrobenzol.

Geeignete 2-Hydroxycarbonsäureester sind beispielsweise: die Methyl-, Äthyl-, Pentyl-, Propyl-, Isopropyl-, Butyl-, sek.-Butyl-, tert.-Butyl- und Isobutylester von Hydroxyessigsäure, 2-Hydroxypropionsäure, 2-Hydroxybuttersäure, 2-Hydroxyvaleriansäure, 2-Hydroxycapronsäure, 2-Hydroxy-2-methylpropionsäure, 2-Hydroxy-2-methylbuttersäure, 2-Hydroxy-2-vinylpropionsäure, 2-Hydroxy-2-phenylpropionsäure; entsprechende Ester der 2-Hydroxyphenylessigsäure, 2-Hydroxy-2-äthylphenylessigsäure, 2-Hydroxy-2-vinylphenylessigsäure; entsprechende Ester der vorgenannten Säuren, die neben der 2-Hydroxygruppe in 2-Stellung noch eine Methoxymethyl-, Äthoxymethyl-, Propoxymethyl-, Isopropoxymethyl-, Butoxymethyl-, Isobutoxymethyl-, sek.-Butoxymethylgruppe tragen; bevorzugt sind die Methyl-, Äthyl-, Propyl-, Isopropyl- und Isobutyl-ester der 2-Hydroxy-2-vinylpropionsäure, 2-Hydroxy-2-methylpropionsäure und 2-Hydroxy-2-methoxymethylpropionsäure.

Die Umsetzung wird im allgemeinen bei einer Temperatur von 80 bis 250, vorzugsweise von 100 bis 220° C, insbesondere bei 130 bis 200° C, unter Druck, vorzugsweise bei 50 bis 1000, insbesondere 80 bis 500, vorteilhaft 80 bis 300 bar, kontinuierlich oder diskontinuierlich durchgeführt. Zweckmäßig verwendet man unter den Reaktionsbedingungen inerte Lösungsmittel. Als Lösungsmittel kommen z. B. in Frage: aromatische Kohlenwasserstoffe, z. B. Toluol, o-, m-, p-Xylol, Methylnaphthalin, Chlorbenzol, o-, p- und m-Dichlorbenzol, o-, m-, p-Chlortoluol; Äther, z. B. Äthylpropyläther, n-Butyläthyläther, Di-n-butyläther, Diisobutyläther, Diisoamyläther, Diisopropyläther, Anisol, Phenetol, Diäthyläther, Äthylenglykoldimethyläther, Tetrahydrofuran, Dioxan; und entsprechende Gemische. Zweckmäßig verwendet man das Lösungsmittel in einer Menge von 400 bis 10 000 Gewichtsprozent, vorzugsweise von 500 bis 2500 Gewichtsprozent, bezogen auf Ausgangsstoff II.

Die Umsetzung wird in Gegenwart einer basischen Verbindung, vorteilhaft in einer Menge von 1 bis 30, vorzugsweise von 5 bis 20 Gewichtsprozent basischer Verbindung, bezogen auf Ausgangsstoff II, durchgeführt. Bevorzugte basische Verbindungen sind Erdalkali- und Alkalisalze von organischen Carbonsäuren oder von Phosphorsäuren und insbesondere tertiäre Amine und cyclische Amidine sowie entsprechende Gemische. Als tertiäre Amine kommen aliphatische, cycloaliphatische, araliphatische, aromatische und heterocyclische Amine in Betracht. Es kommen z. B. als basische Verbindungen in Frage: Magnesiumacetat, Natriumformiat, Natriumacetat, Natriumpropionat, Natriumbutyrat, Kaliumformiat, Kaliumacetat, Kaliumpropionat, Kaliumbutyrat, Natriumphosphat, Calciumphosphat, Magnesiumphosphat, Kaliumphosphat; Trimethylamin, Triäthylamin, Tripropylamin, Triisopropylamin, Tributylamin, Triisobutylamin, Tri-sek.-butylamin, Tri-tert.-butylamin, Tribenzylamin, Tricyclohexylamin, Tri-tert.-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyltoluidin, N,N-Dipropyltoluidin, N,N-Dimethyl-p-aminopyridin, N,N-Diäthyl-p-aminopyridin, N,N-Dipropyl-p-aminopyridin, N-Methylpiperidin, N-Äthylpiperidin, N-Methylpyrrolidin, N-Äthylpyrrolidin, N-Methylimidazol, N-Äthylimidazol, N-Methylpyrrol, N-Äthylpyrrol, N-Methylmorpholin, N-Äthylmorpholin, N-Methylhexamethylenimin, N-Äthylhexamethylenimin, Pyridin, Chinolin, Isochinolin, α-Picolin, β-Picolin, γ-Picolin, Isochinolin, Pyrimidin, Acridin, N,N,N',N'-Tetramethyläthylendiamin, N,N,N',N'-Tetraäthyläthylendiamin, N,N,N',N'-Tetramethyl-hexamethylendiamin, Chinoxalin, Chinazolin, N-Propyldiisopropylamin, N,N-Dimethylcyclohexylamin, 2,6-Lutidin, 2,4-Lutidin. Bevorzugt sind Pyridin, Chinolin, Isochinolin, Toluidin, p-Dimethylaminopyridin, Lithiumacetat, Natriumacetat oder Kaliumacetat und insbesondere 1,4-Diaza-bicyclo-2,2,2-octan

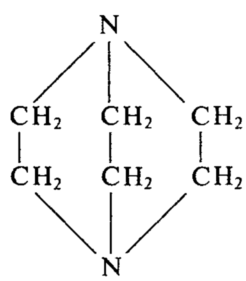

N-Methylimidazol, 1,5-Diaza-bicyclo-[4,3,0]-non-5-en

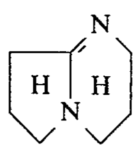

und 1,5-Diaza-bicyclo-[5,4,0]-undec-5-en

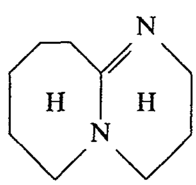

Der Selenkatalysator kann Selen in verschiedener Modifikation, z. B. in Gestalt von amorphem, glasigem, hexagonalem, monoklinem, kubischem Selen, vorteilhaft in Gestalt von Selenpulver, enthalten. Zweckmäßig ist pulverförmiges bzw. amorphes bzw. feinverteiltes Selen, wie es beispielsweise bei der Aufarbeitung von selenhaltigem Bleikammerschlamm oder beim Abrösten selenhaltiger Sulfide anfällt.

Das nach den üblichen Verfahren hergestellte Kohlenmonoxid ist in reinem und meist schon in rohem Zustand für das erfindungsgemäße Verfahren geeignet. Zum Beispiel kann das aus der Direktsynthese aus Koks und Sauerstoff (Otto-Verfahren); aus der Abtrennung aus Synthesegasen, Generatorgas, Wassergas, Verkokungsgas, Verschwelungsgas, Gasgemischen der Steinkohle- bzw. der Koksvergasung; gewonnene und in üblicher Weise, z. B. mit der Kupferlaugenwäsche, dem Cosorbverfahren, der Flüssig-Methanwäsche, der Tieftemperaturtrennung, aufgearbeitete rohe Kohlenmonoxid verwendet werden. Es kann noch Verunreinigungen wie Kohlendioxid, Sauerstoff, Wasserstoff, Methan, Stickstoff, Argon enthalten, wobei zweckmäßig die Gesamtmenge der

Verunreinigungen an Kohlendioxid, Sauerstoff, Methan und insbesondere Wasserstoff 0,5 Gewichtsprozent des Kohlenmonoxids nicht übersteigt. Der erfindungsgemäße höhere Reaktionsdruck wird zweckmäßig durch den Druck des eingeleiteten Kohlenmonoxid zusammen mit dem Eigendruck der übrigen Reaktionsteilnehmer bei der gewählten Reaktionstemperatur eingestellt, gegebenenfalls können zur Druckeinstellung auch unter den Reaktionsbedingungen inerte Gase, z. B. Stickstoff, verwendet werden. Zweckmäßig kommen 10 bis 100, insbesondere 15 bis 50 Mol Kohlenmonoxid, bezogen auf Ausgangsstoff II, in Frage.

Die Reaktion kann wie folgt durchgeführt werden: Ein Gemisch von Ausgangsstoff II, III, Kohlenmonoxid, Katalysator, Lösungsmittel und basischer Verbindung wird während 1 bis 4 Stunden bei der Reaktionstemperatur und dem Reaktionsdruck gehalten. Dann wird der Endstoff aus dem Gemisch in üblicher Weise, z. B. durch Filtration, Destillation und Umkristallisation des Rückstands, abgetrennt.

Die nach dem Verfahren der Erfindung erhältlichen bekannten und neuen N-Aryloxazolidin-2,4-dione der Formel I sind wertvolle Wirkstoffe und Ausgangsstoffe für die Herstellung von Pflanzenschutzmitteln, Farbstoffen und Pharmazeutika. Bezüglich der Verwendung wird auf die vorgenannten Veröffentlichungen verwiesen.

Die in den folgenden Beispielen genannten Teile sind Gewichtsteile. Die Volumenteile verhalten sich zu den Gewichtsteilen wie Gramm zu Liter.

## Beispiel 1

35 Teile 2-Hydroxy-2-vinylpropionsäureisobutylester, 9,6 Teile 3,5-Dichlornitrobenzol, 1 Teil Selen und 1 Teil 1,4-Diaza-bicyclo-[2,2,2]-octan

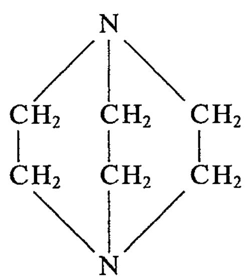

werden zusammen mit 100 Teilen Chlorbenzol in einen 400 Teile fassenden Autoklaven gegeben. Der Autoklav wird dreimal mit Stickstoff gespült, anschließend wird Kohlenmonoxid bis zu einem Anfangsdruck von 150 bar aufgepreßt. Die Reaktionsmischung wird 3 Stunden auf 190° C erwärmt. Danach wird abgekühlt, das Kohlenmonoxid durch Stickstoff verdrängt und die Reaktionslösung aus dem Autoklaven abgelassen. Nach Filtration des Gemisches destilliert man Lösungsmittel, überschüssigen 2-Hydroxy-2-vinylpropionsäureisobutylester und nicht umgesetztes 3,5-Dichlornitrobenzol ab und gibt den Rückstand in 25 Teile Methanol. Nach Filtration erhält man 6,5 Teile N-(3',5'-Dichlorphenyl)-5-methyl-5-vinyloxazolidin-2,4-dion (91% der Theorie) vom Fp. 108 bis 110° C. Der Umsatz an 3,5-Dichlornitrobenzol beträgt 50 Prozent.

## Beispiel 2

Analog Beispiel 1 werden 9,6 Teile 3,5-Dichlornitrobenzol mit 56 Teilen 2-Hydroxy-2-methoxymethylpropionsäureäthylester, ein Teil Selen und ein Teil 1,4-Diaza-bicyclo-[2,2,2]-octan in 100 Teilen Chlorbenzol bei 200 bar Anfangsdruck und 190° C 3 Stunden zur Reaktion gebracht. Nach Filtration des Gemisches destilliert man Lösungsmittel und überschüssigen 2-Hydroxy-2-methoxymethylpropionsäureäthylester ab und gibt den Rückstand in 25 Teile Methanol. Nach Filtration erhält man 13,2 Teile N-(3',5'-Dichlorphenyl)-5-methoxymethyl-5-methyloxazolidin-2,4-dion (87% der Theorie) vom Fp. 105 bis 108° C. Der Umsatz an 3,5-Dichlornitrobenzol ist praktisch quantitativ.

**Patentanspruch**

Verfahren zur Herstellung von N-Aryloxazolidin-2,4-dionen der Formel

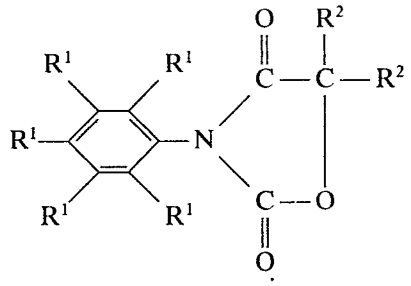

(I)

worin die einzelnen Reste $R^1$ und $R^2$ gleich oder verschieden sein können und jeweils ein Wasserstoffatom, einen aliphatischen, cycloaliphatischen, araliphatischen oder aromatischen Rest bedeuten, die beiden Reste $R^2$ darüber hinaus zusammen auch einen Methylenrest bezeichnen, $R^1$ auch jeweils für ein Halogenatom oder den Rest $-K-R^3$ steht, $R^3$ einen aliphatischen Rest bezeichnet, X ein Sauerstoffatom oder ein Schwefelatom bedeutet, dadurch gekennzeichnet, daß man ein Nitrobenzol der Formel

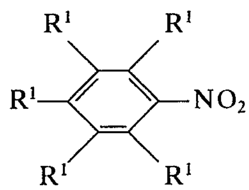

(II)

worin $R^1$ die vorgenannte Bedeutung besitzt, mit einem 2-Hydroxycarbonsäureester der Formel

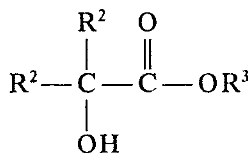

(III)

worin $R^2$ und $R^3$ die vorgenannte Bedeutung besitzen, mit Kohlenmonoxid in Gegenwart von Selen und einer basischen Verbindung bei erhöhtem Druck und erhöhter Temperatur umsetzt.

**Claim**

A process for the preparation of an N-aryloxazolidine-2,4-dione of the formula

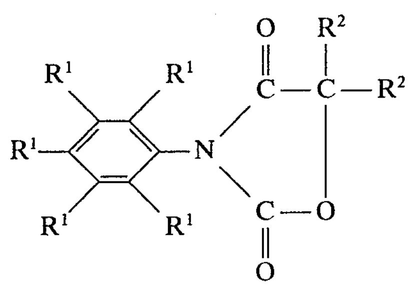

(I)

where the individual radicals $R^1$ and $R^2$ may be identical or different and each is hydrogen or an aliphatic, cycloaliphatic, araliphatic or aromatic radical, the two radicals $R^2$ may also together be a methylene radical and $R^1$ may also be halogen or $-X-R^3$, where $R^3$ is an aliphatic radical and X is oxygen or sulfur, characterized in that a nitrobenzene of the formula

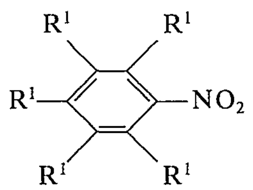

(II)

where $R^1$ has the above meaning, is reacted with a 2-hydroxycarboxylic acid ester of the formula

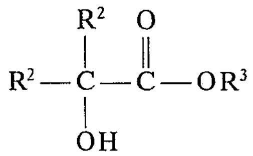

(III)

where $R^2$ and $R^3$ have the above meanings, and with carbon monoxide in the presence of selenium and a basic compound under superatmospheric pressure at an elevated temperature.

**Revendication**

Procédé de préparation de N-aryl-oxazolidine-2,4-diones de la formule

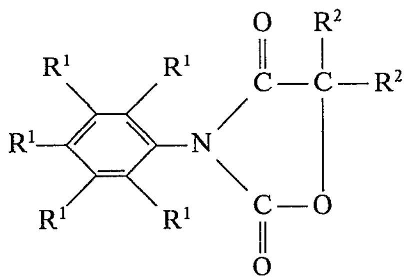

(I)

dans laquelle les divers restes $R^1$ et $R^2$ peuvent être identiques ou différents et désignent chacun un atome d'hydrogène ou un groupe aliphatique, cycloaliphatique, araliphatique ou aromatique, les deux restes $R^2$ pouvant en outre former ensemble un groupe méthylène et $R^1$ représenter un atome d'halogène ou un groupe $-X-R^3$, dans lequel X représente un atome d'oxygène ou de soufre et $R^3$ un groupe aliphatique, caractérisé en ce que l'on fait réagier un nitrobenzène de la formule

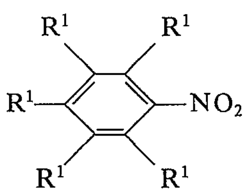

(II)

dans laquelle $R^1$ possède la signification définie, sous pression et à température accrues, en présence de sélénium et d'un composé basique, avec un ester d'acide 2-hydroxycarboxylique de la formule

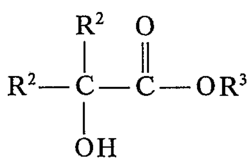

(III)

dans laquelle $R^2$ et $R^3$ possèdent les significations définies, et de l'oxyde de carbone.